# EUROPEAN PATENT APPLICATION

(11) **EP 0 627 243 A1**
(43) Date of publication of application: **07.12.1994**
(21) Application number: 94303339.9
(22) Date of filing: 09.05.1994
(51) Int. Cl.: A61N 5/06

(54) **Laser phototherapy**

(30) Priority: 24.05.1993 US 36568
(71) Applicant: OHMEDA INC., Liberty Corner, New Jersey 07938-0804 (US)
(72) Inventor: Kremenchugsky, Vladimir, Reisterstown, Maryland 21136 (US)
(74) Representative: Gough, Peter

(57) **Abstract**

A phototherapy system and method for providing light therapy to reduce the bilirubin level in infants wherein a laser (78) is used as the source of light radiation. The light radiation from the laser is transmitted at a predetermined wavelength through an optical fiber (82) to a fiberoptic pad where it radiates toward the infant. In one embodiment, a plurality of infants may be treated through a plurality of fiberoptic pads (88), all receiving the light radiation from a common laser. In such embodiment, a control means (94,96,98,100) is also provided to detect the intensity of light impinging upon the infant and using that value of intensity to control the light radiation transmitted to the fiberoptic pad.

## Description

This invention relates to a system for providing phototherapy to an infant, and, more particularly, to a fiberoptic light pad that is used to provide light to the infant and which has, as its source of light radiation, a laser.

Hyperbilirubinemia is an affliction of new-born infants typified by an elevated level of a toxic molecule known as bilirubin in the infant's blood.

Bilirubin is normally conjugated by the liver and excreted in the bile, however, at the period immediately after birth, the new-born's liver is not sufficiently active to conjugate bilirubin and the level of bilirubin therefore, builds up in the blood causing a form of jaundice.

Treatment is needed and if the bilirubin is extremely high, that treatment may require a blood exchange transfusion. At lower hyper-levels, however, the typical treatment is by phototherapy which is believed to cause a photochemical reaction in the bilirubin by changing the bilirubin to lumirubin which is eliminated by the infant through excretion in the urine and bile.

Most phototherapy at present is by means of banks of lights that are directed toward the infants skin. While effective, the lights are somewhat cumbersome in that they interfere with personnel attending to the infant and generate undesirable heat surrounding those personnel.

A more recent commercial means of combating hyperbilirubinemia is by means of a light blanket, typical of which is made of woven fiberoptics as shown and described in Daniel U.S. Patent 4,234,907. Certain wavelengths of light are considered to be optimum for phototherapy and those wavelengths fall within the range generally of 440-540 nanometers. Accordingly, current fiberoptic blankets are powered from a conventional light source, such as a halogen resistance type bulb and which includes a reflector to concentrate the beam of light generally in the proper direction and further includes an optical glass filter to filter the beam of light to the desired wavelength range i.e. 440 - 540nm. range.

One of the difficulties of such an arrangement, however, is that conventional light bulbs degrade with time and therefore the dosage of light therapy given to the infant may vary over the lifetime of the bulb. Also, the emission of the bulb drifts with time and therefore frequent recalibration of bulb emission is required. Typically, lifetimes of resistance type bulbs are also fairly short and may range from about 500 to 800 hours. Obviously, therefore, the resistance bulbs need frequent replacement which is not only bothersome but costly in terms of the time wasted by hospital personnel.

In addition, the current filters, while able to select a range of wavelengths, are not narrow and therefore a fairly wide range of wavelengths is transmitted to the infant. Some of such wavelengths are not helpful, and indeed may be harmful, but in any case, the physician is unable to exert any control over the wavelengths. There is some evidence that different infants may be aided by certain wavelengths to reduce bilirubin at a more rapid pace than other wavelengths within the current range of wavelengths administered to the infant through current filters.

The conventional resistance bulbs, as stated, require glass filters to select the range of wavelengths that do alleviate bilirubin and such filters absorb or reflect about 95% of the radiation energy, thus the entire system is caused to operate at highly elevated temperatures. Adding to the heat problem is the need for higher wattage bulbs to compensate for the losses incurred through the filter. Higher temperatures not only create problems from an insulation standpoint, but also affect the reliability of the unit and the lifetimes of the bulbs.

As a further detrimental effect of the overheating problem, the elevated temperatures change the spectral and optical characteristics of many optical filters and thus, again, the wavelength actually applied to the infant may change over time as may the intensity of the light radiation.

The treatment afforded the individual infant is, therefore, somewhat uncertain and incapable of precise dosage.

Further large optical losses in conventional units are incurred in transmitting the light radiation from the resistance bulb to the fiber optic cable. Since the beam of light from a common resistance bulb is non-directional and non-parallel to the fiber optic cable walls, it becomes difficult to align the light bulb, filter and cable to transmit efficiently as possible the irradiated energy from the bulb to the fiberoptic cable.

As an example, therefore, in the current commercial product, using a halogen resistance bulb rated at 140 watts, the actual light density measured at the infant from a fiberoptic pad of about 22.0 square inches, is about 15-35 microwatts per square centimeter. Certainly more light density is desirable without, of course, the attendant problems relating to heat, however, with the current resistance bulbs, higher light densities are extremely difficult to attain.

Therefore, all of the above disadvantages cause severe limitations in the use of resistance bulbs, including not only changes in the spectral range and intensities of delivered light radiation, but, by principle, create an overall limit to the level of intensity of light that can be administered to the infant for phototherapy. Since the rate of bilirubin decline is proportional to light intensity, the efficacy of phototherapy is reduced.

According to a first aspect of the present invention, a phototherapy system for providing light radiation to an infant having at least one fiberoptic pad adapted to be placed near the infant to allow light emitted from the pad to impinge upon the patient, comprises a light source for emitting light radiation and a fiber optic coupling means for transmitting light from said light source to said fiberoptic pad and characterised in that said light source comprises a laser producing a monochromatic light at a known wavelength.

According to a second aspect of the present invention, a system for providing phototherapy to infants is characterised by:
a) a laser providing coherent light radiation at a predetermined wavelength;
b) a plurality of fiberoptic light pads adapted to be placed near the infant;
c) a plurality of optical fibers connecting said laser to each of said plurality of fiberoptic light pads such that the light radiation from said laser is transmitted outwardly from said fiberoptic light pads to impinge upon the infants;
d) light sensors for detecting the intensity of light emitted by each of said fiberoptic light pads toward the infants and to produce an electrical signal representing such light intensities; and
e) control means adapted to respond to the electrical signal from each of said light sensors to control the intensity of light transmitted individually to each of said plurality of fiberoptic pads to thereby control the intensity of light impinging upon the infants from the fiberoptic pads for providing phototherapy.

According to a further aspect of the present invention, a method of providing light therapy to an infant comprises the steps of:
a) providing a light emitting fiberoptic pad;
b) locating the fiberoptic pad adjacent an infant such that light eminating from the pad impinges upon the infant;
c) directing light radiation from a laser into the fiberoptic pad to cause it to emit light radiation of a predetermined monochromatic wavelength upon the infant.

According to yet a further aspect of the present invention, a method of providing light radiation to a plurality of infants for phototherapy, comprises the steps of:
a) providing a plurality of fiberoptic woven phototherapy pads;
b) placing each of said plurality of phototherapy pads in position in close proximity to an infant;
c) directing light from a single laser having a known monochromatic wavelength into each of said plurality of phototherapy pads to cause light radiation to be emitted from each of the phototherapy pads to impinge upon the infants;
d) sensing the intensity of the light radiation emitted from each of said plurality of phototherapy pads and impinging upon the infants; and
e) controlling the intensity of the light emitted from each of said plurality of phototherapy pads in response to the intensity sensed in step d).

In accordance with the present invention, a fiberoptic light pad is utilised to alleviate bilirubin in an infant wherein the system includes a high intensity, monochromatic light source, such as a laser, as the source of light radiation and includes a means to control the intensity of the applied light radiation.

The system of the present invention utilises a fiberoptic pad, fiberoptic cable transmitting light to such pad, a laser as a source of the radiation and a special connector to connect the laser source to the fiber optic cable and avoid optical losses. One of the advantages of utilizing a laser as a source of radiation to alleviate bilirubin is the availability of coherent, monochromatic radiation at a specific, chosen wavelength. Thus, the wavelength may be chosen specially for the application and in the case of a tunable laser, the coherent source may not only be selected by the physician but may be changed by the physician to vary the depth of the penetration of the light within the infants skin.

Also, with a laser as the source of light radiation for phototherapy using a fiberoptic pad, the power available, and thus the intensity of the light radiation actually applied to the infant is considerably higher without encountering the overheating problems associated with resistance bulbs.

The aforementioned Daniel patent includes a reference to a fiberoptic pad having a laser as a source of infrared energy for heating (Col. 6, lines 31-37) but did not suggest that light radiation from a laser could or would be applicable for phototherapy for an infant.

As an example of the effect of the intensity of the light radiation upon the reduction of bilirubin in infants, a study was conducted and reported in an abstract of a paper presented at The American Paediatric Society, Society of Paediatric Research at New Orleans, LA in May of 1991 entitled, Bright Light is The Right Light: Multicenter Trial of a Novel Phototherapy Device in which the results showed the efficacy of phototherapy was improved by increasing the intensity of the light radiation applied to the infant.

A further study of the effect of the wavelength of the phototherapy on its efficacy in reducing the bilirubin level in infants was reported in Clinics in Perinatology, Vol. 17, No. 2, June 1990. Thus, various studies undertaken and continuing to date have investigated both the intensity of the level of phototherapy and the effect of various differing wavelengths of such light radiation and certain conclusions reached. As a research tool, therefore, the present invention utilizing a laser is of incalculable assistance in that high intensities are readily achievable (without filters) and a wide range of wavelengths of monochromatic light radiation can be applied, even from the same laser, to the infant to ascertain the therapeutic effect.
Losses are also minimised with the utilisation of a laser inasmuch as the radiation emanating from a laser is directional and parallel and therefore the laser radiation can be directed towards a coupling device to match its output with that of the fiberoptic cable.

In addition, precise radiation dosage control is possible with a pulse laser by changing the on-off cycle or by modifying the waveform of the pulses. The laser itself may be of a commercially available variety and may be continuous wave or pulsed wave. Many of the commercially available lasers provide radiation in the wavelength range that is effective for the alleviation of bilirubin and therefore filters are unnecessary. As such, the attendant radiation losses associated with filters is also eliminated. Thus, the transmission of energy from the laser to the fiberoptic pad is more efficient than through the use of resistance bulbs.

Preferably, a laser is chosen that has tunable power, that is the power is selectable over a range of settings. Even more preferably, the laser chosen is of the type that has a plurality of selectable wavelengths so that the physician may choose the appropriate wavelength or change that selection during the therapy to achieve better results.

As a further embodiment, multiple fiberoptic pads may be used when the source of radiation is a laser inasmuch as considerably more power is available than is available from conventional resistance bulbs and the power losses are greatly reduced in the optical system that transmits that light radiation from a laser to the fiberoptic pad or pads. Thus, not only may multiple fiberoptic pads be used on an individual infant, but a plurality of pads may be used in a nursery for many infants, all operating off a central laser. Eventually, systems may be developed wherein the single source of optical radiation from a laser may be piped to plug-in outlets or stations throughout a nursery or suite of rooms where phototherapy is being used on infants.

When a single laser is used as a source for multiple infants, however, it is also necessary to include a control system to control the light reaching the individual infant since a change of radiation applied to one infant could have an effect on other infants. Thus, when a plurality of infants is being administered phototherapy from a common laser, a system is provided that allows individual fiberoptic pads to be varied in intensity by the attending personnel, or turned off and on, without changing the desired set intensity of phototherapy applied to other infants. This is accomplished by a feedback system that senses the amount of light administered by each individual fiberoptic pad and provides control based on that sensed intensity of light.

The light sensor may be various types of light sensors.

An embodiment of the invention will now be described, by way of example, reference being made to the Figures of the accompanying diagrammatic drawings in which:
Figure 1 is a schematic view of a fiberoptic pad phototherapy system constructed in accordance with the present invention;
Figure 2 is a side cross-sectional view of a coupler used with the present invention;
Figure 3A and Figure 3B are schematic views showing an embodiment of the present invention having multiple fiberoptic pads;
Figure 4 is a schematic view of a further embodiment in which a plurality of fiberoptic pads are used having a control scheme to control the intensities of the pads;
Figure 5 is an example of a fiberoptic detector usable in the control scheme of Figure 4; and
Figure 6 is a further example of a fiberoptic detector usable with the control scheme of Figure 4.

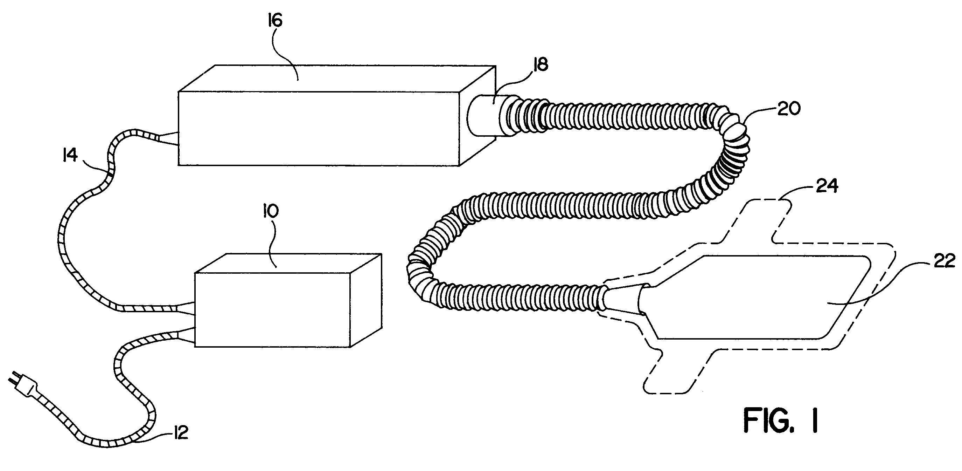

Turning first to Figure 1, there is shown a schematic view of a phototherapy system constructed in accordance with the present invention and including a power supply 10 adapted to be connected to a standard electrical service by power cord 12. Power supply 10 converts the standard voltage available, for example, in most hospitals to the voltages required by the present system. In general, although the power supply is shown separately in Figure 1, the individual power supplies can be supplied along with the particular laser by the same manufacturer. That power is thus transmitted, again by a standard cable 14 to laser illuminator 16 which includes the laser for operating the phototherapy system as will be explained. A coupler 18 is provided at the output of the laser illuminator 16 and which couples the radiant energy generated by the laser illuminator 16 to one end of a fiberoptic cable 20.

The fiberoptic cable 20 transmits the light radiation received from laser illuminator 16 to the fiberoptic pad 22 where that radiation can be applied to an infant. A disposable cover 24 may also be provided to cover the fiberoptic pad 22 and which is discarded after each use and which protects the fiberoptic pad 22 from contamination.

As to the lasers usable with the present invention, various lasers are commercially available that have an emission radiation in the desired range of wavelengths, that is in the blue-green range (400-520 nm.). One preferred laser is a tunable Argon Ion laser available from the Omnicrome Corporation of Chino, CA as Model 532-MAP and which has eight selectable wavelengths and tunable output power in the range of from about 1.5 to 33 mW. With that laser, the physician can not only select the desired wavelength but may also select the power setting depending on the particular application.

With the aforementioned laser from Omnicrome, the wavelengths selectable vary from 454 nm. to 514 nm. and include eight wavelengths that may be chosen, all of which are within the range acceptable for phototherapy for an infant. Taking, for example, the 488 nm. wavelength, the output power may be tuned from about 1.5 milliwatts to about 33 milliwatts. By using that variable power, the light density that can actually be provided to the infant, using the standard current commercial woven phototherapy pad of 22.0 square inches ranges from about 10 to about 220 microwatts per square centimeter.

Other lasers suitable for the present invention are Dye lasers available from Coherent Inc. of Palo Alto, CA; Dye, Argon-ion lasers available form Spectra-Physics Laser Inc. of Mountain View, CA.; as well as various other suppliers of lasers such as Dye, Argon; Dye, nitrogen; Dye, eximer/YAG; Dye,eximer/YAGNd/Cu; Helium-Cadmium; Argon-Krypton and Laser Diodes.

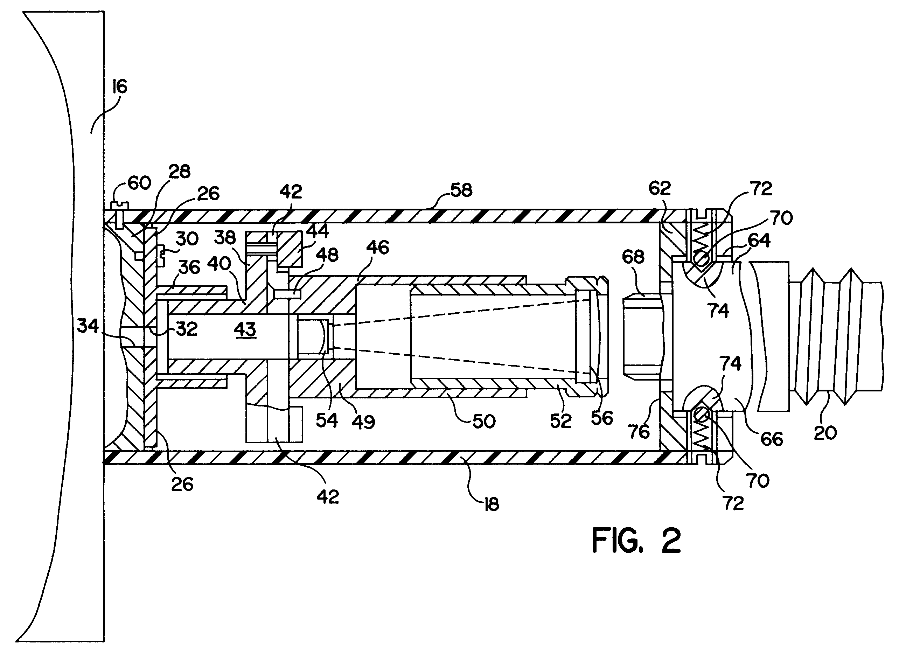

Turning now to Figure 2, there is shown a side cross-sectional view of coupler 18 usable with the present invention. Again, couplers for lasers are commercially available and current suppliers are NSG America, Inc. of Somerset, N.J. and Oriel Corp. of Stratford, CT.

As a typical coupler, Figure 2 includes a coupler adapter 26 that fits over an accessory mount 28 projecting outwardly from laser illuminator 16. Coupler adapter 26 may be secured to accessory mount 28 by means such as screws 30 (only one of which is shown) and has an opening 32 aligned with the cylindrical opening 34 through which the light radiation emanates from laser illuminator 16. As indicated, the light radiation is coherent and is directed along the longitudinal axis of the cylindrical opening 34.

Typical diameters of the light radiation from laser illuminator may be in the range of 0.7mm. for Dye lasers from Coherent, Inc; 0.54mm. for Dye, Argon-ion lasers from Spectra-Physics Laser Inc. to 3.5mm. for Argon-Krypton lasers from American Laser Corp. of Salt Lake City, UT. Obviously, those diameters are too small for the intended use as will become apparent in the following description and thus, the coupler 18 is used to shape and expand the diameter of the light radiation to the desired size.

Coupler adapter 26 further includes a cylindrical flange 36 extending outwardly therefrom and which is internally threaded. An adjustable plate 38 is mounted to the cylindrical flange 36. Adjustable plate 38 comprises a mounting flange 40 that is threadedly engaged to the internal threads of cylindrical flange 36. A plate 42 is mounted to the mounting flange 40 and is adjustable with respect to mounting flange 40 by means of knurled cap screws 44, generally three in number, and which allow adjustment of the position of plate 42 with respect to mounting flange 40 to provide alignment of the optical components to be described. A cylindrical passage 43 is formed in adjustable plate 38 to allow the passage of the light radiation from laser illuminator 16.

A laser beam expander 46 is mounted by means of screws 48 (only one of which is shown) to adjustable plate 38 and comprises a main body 49 having an outwardly extending cylindrical flange 50 into which is fitted an inner cylindrical flange 52. A diverging lens 54 is secured within cylindrical flange 50 and which diverges the otherwise relatively small diameter light radiation emanating from laser illuminator 16. A converging lens 56 is affixed within inner cylindrical flange 52 and which receives the diverging beam of light radiation and reforms it to the desired diameter and shapes the beam back into a direct beam that travels coaxial with the longitudinal axes of cylindrical flange 50 and inner cylindrical flange 52. Obviously, by axially moving the inner cylindrical flange 52 with respect to cylindrical flange 50, the focus (diameter) of the beam of light radiation emanating from converging lens 56 may be adjusted as desired.

As described, beam expanders and adjustable plates are commercially available and which fit on to various lasers to expand the light radiation beam from the laser to a larger, focusable beam of radiation. One of such beam expanders is available from Oriel Company of Stratford, CT. and which are specially designed to interfit to lasers available from that company. The typical optical losses experienced with currently available laser beam expanders do not exceed 10%.

A cover 58 surrounds the laser beam expander 46 and is secured to accessory mount 28 by means such as screw 60. At the end of the cover 58 extending away from laser illuminator 16, there is positioned a fiberoptic connector holder 62 which is firmly affixed to cover 58. Fiberoptic connector holder 62 has an opening 64 formed therein for receiving a fiberoptic connector 66 of fiberoptic cable 20. Fiberoptic connector 66 is of standard commercial design and includes a stub 68 that crimps the plurality of optical fibers and has its end facing laser beam expander 46 smoothed with the optical fiber ends within stub 68 contained generally parallel to each other and coaxial with the fiber optic cable 20. The fiberoptic connector 66 may be releasably secured to fiberoptic connector holder 62 through means such as a plurality of balls 70 that are biased by springs 72 into suitable recesses 74 in fiberoptic connector 66.

An internal flange 76 on fiberoptic connector holder 62 provides a stop for the inward movement of the fiberoptic connector 66 and thus positions the fiberoptic connector 66 in a known fixed position with respect to the laser beam expander 46. As such, therefore, the fiberoptic connector 66 may be readily disengaged and reengaged with fiberoptic connector holder 62 without changing the overall optics of the system. An interlock, not shown, can be provided such that power to the laser is shut off whenever the fiberoptic connector 66 is disengaged from fiberoptic connector holder 62. Thus the system can safely be used without the danger of light radiation from the laser emanating to the outside and possibly reaching personnel.

As can now be seen, therefore, the laser illuminator 16 provides light radiation in a straight coaxial direction and which beam of radiation is expanded and projected on the receiving end of the fiberoptic connector 66. The beam of radiation is directed in a straight path toward such end and therefore an efficient coupling of the radiation is accomplished. By selecting one of various commercially available lasers, the proper wavelength or selection of wavelengths may be made available by that laser for transmission through a fiberoptic cable to the fiberoptic pad for administering light therapy to an infant.

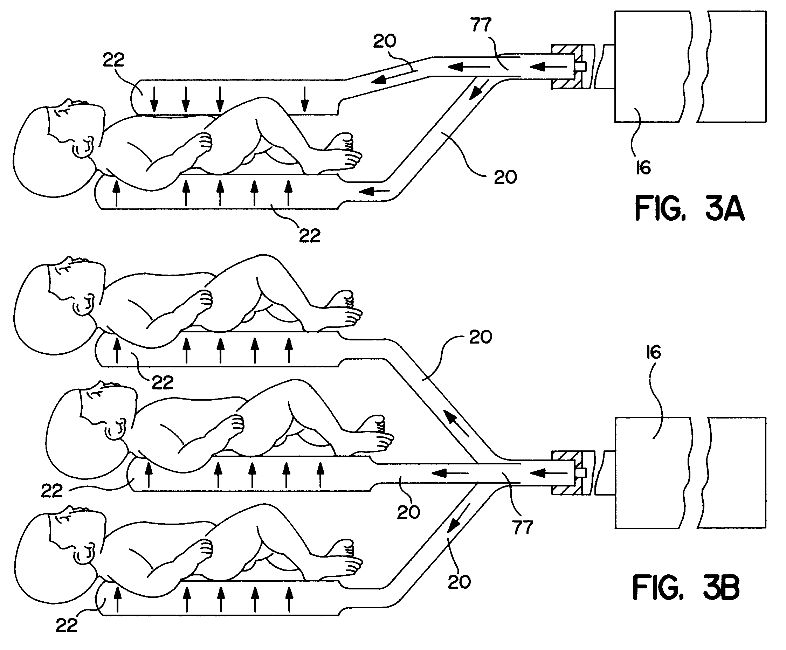

Turning now to Figures 3A and 3B, there is shown a schematic view of an alternate embodiment that may be employed through use of the present invention. Since one of the advantages of utilizing a laser as the source of light radiation for phototherapy is its ability to minimise losses and to provide considerably more efficient energy to the phototherapy pad, a multiple number of fiberoptic pads 22 may be utilised. In particular, in Figure 3A, a single infant is receiving phototherapy from a plurality of fiberoptic pads 22 and in Figure 3B, the same laser illuminator 16 is utilised to provide phototherapy to a plurality of infants, each having a separate fiberoptic pad 22. A light splitter 77, again readily available commercially, is included in the Figure 3 embodiments.

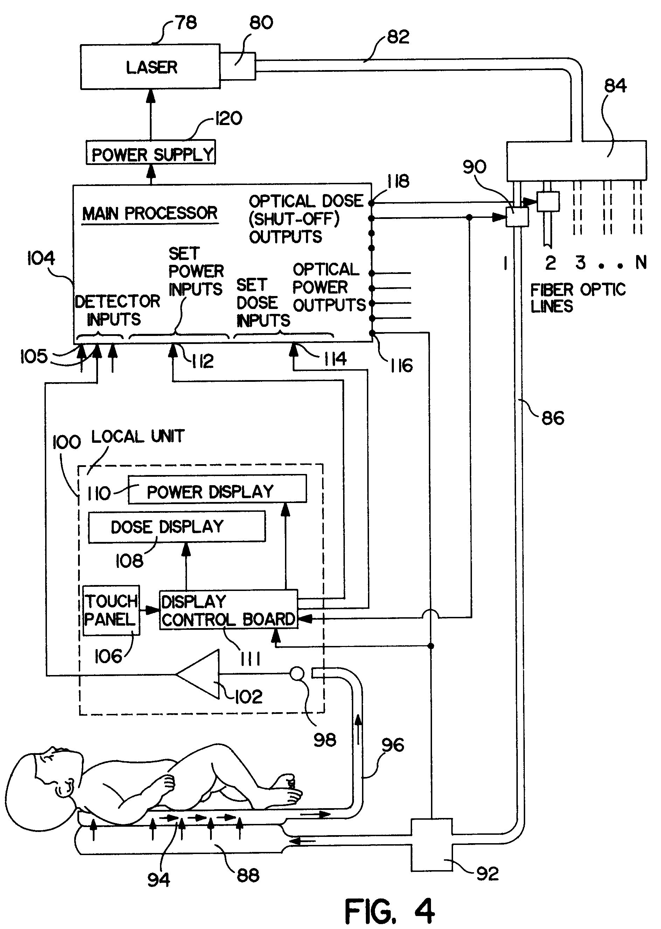

Turning now to Figure 4, there is shown a schematic of a phototherapy control scheme that is usable where a plurality of fiberoptic pads are administering phototherapy to a plurality of infants using a single laser as the source of light radiation. In such case, a powerful Argon laser is preferred (up to 20 watts or more) and such lasers are available from American Laser Corp. of Salt Lake City, Utah, specifically, its Model 930.

In Figure 4 a laser 78 directs high intensity monochromatic light radiation through a beam expander 80 and thus through a optical fiber 82 (referred to as an optical fiber for brevity, however the optical fiber 82 is normally a plurality of bundled optical fibers) where the light radiation is introduced into a multichannel fiber optic splitter 84 where the light radiation is split into a plurality of channels, up to a quantity of n. Such splitters are available commercially and one such supplier is the Oriel Corp., Model 77536 trifurcated fiber optic bundles or alternately from Mitsubishi Gas Chemical America, Inc. of New York, N.Y. or Newport Co. of Irvine, CA.

Since the further description of the system would be the same for all n channels, only one of the channels will be explained in detail.

Taking a representative channel, therefore, an optical fiber 86 carries the light radiation emanating from the multichannel fiberoptic splitter 84 to the fiberoptic light pad 88.

An optical shutter 90 is located in the optical fiber 86 and controls the on-off status of light radiation through optical fiber 86. Such laser shutters are also commercially available for wavelengths from X-ray to far infrared from NM Laser Products, Inc. of Sunnyvale, CA. Thus, the optical shutter 90 is actuated by an electrical signal to switch between an off position where the light radiation is blocked and thus cannot reach fiberoptic mat 88 and an on position where the light radiation travels unimpeded through optical fiber 86.

Also included in the optical fiber 86 is a variable attenuator 92 and which is used to control the degree of light radiation that passes through optical fiber 86 to fiberoptic light pad 88. Again, suitable variable attenuators are commercially available from I.D.S. Fitel, Inc of Ontario, Canada.

A light sensor 94 is positioned adjacent the fiberoptic light pad 88 and senses the light output of fiberoptic light pad 88 that reaches or is directed toward the infant for phototherapy. As shown, the light sensor 94 is in the form of a pad positioned intermediate the fiberoptic light pad 88 and the infant. However, various types of light sensors may be used, including those adapted to receive light directly from optical fiber 86 on the infant side of the variable attenuator 92.

Light radiation is therefore sensed by light sensor 94 and which is indicative of the amount of light impinging upon the infant. The sensed light is transmitted via optical fiber 96 to an optical transducer 98 within a local display unit 100 located within close proximity to the infant so as to be accessible to personnel attending to the infant. The functions associated with local display unit 100 are schematically included within the dotted lines in Figure 4.

Optical photo-transducer 98 may be of typical commercial design and which generates an electrical signal in proportion to its received optical light input. Thermal radiation detectors may also be used and such units are available from Laser Precision Corp. of Utica, N.Y. In either case, the electrical signal generated by optical photo-transducer 98 is amplified by amplifier 102 and the signal is thus transmitted to the central processing unit (CPU) 104 as one of the plurality of detector inputs 105.

At the local display unit 100, the user inputs by a touch panel 106 the dosage and the power desired and that value is displayed, respectively in the local display unit 100 by the dose display 108 and power display 110. Control of the dose display 108 and power display 110 are through display control board 111.

As signals are received in the CPU 104 indicative of light impinging upon the infant, that is, from amplifier 102, that signal is compared with the preset values by the user and transmitted to CPU 104 by the display control board 111 as set power inputs 112 and set dose inputs 114. CPU 104 thus controls the variable attenuator 92 via optical power outputs 116 to set the intensity of light directed on the infant and also controls the optical shutter 90 via optical dose outputs 118 to turn off the light radiation when the prescribed amount of radiation has been given to the infant as indicated by the dose display 108. In both instances, the optical power outputs 116 and optical dose outputs 118 also communicate with the display control board 111 to indicate the status of the optical shutter 90 and the variable attenuator 92.

As a further control, CPU 104 analyses the signals from all of the light sensors 94 and regulates the laser power (for example) by changing the electric current operating the laser via power supply 120 in accordance with the overall light power consumption throughout the system.

In the system, therefore, the laser 78 can be used with the needed controls to provide light radiation to a plurality of infants, and the control of each infant is set by a user to whatever dosage and intensity is desired on an individual basis and a change in dosage or intensity to one infant will not affect the dosage or intensity of any of the other infants using the same central laser as the light radiation source.

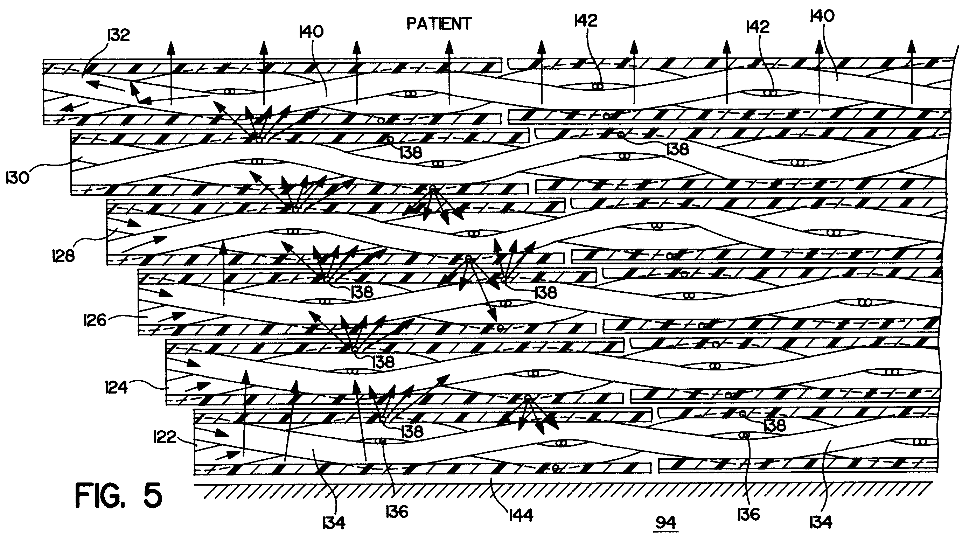

Turning now to Figure 5, there is shown an enlarged schematic fragmentary longitudinal section of a light sensor 94 usable with the present invention. As is more apparent by review of the Daniel U.S. Patent 4,234,907, such fiberoptic pads are multilayer and are manufactured generally in accordance with U.S. Patent 4,907,132, the disclosure of which is incorporated herein by reference.

In Figure 5, a plurality of interwoven layers are shown, each layer being manufactured by means of the disclosure of the aforementioned Daniel U.S. Patent 4,234,907, Those layers are identified in Figure 5 as light emitting layers 122, 124, 126, 128, 130 and light detecting layer 132 where light detecting layer 132 faces the infant. Each of the light emitting layers 122-130 are identical and are the normal layers in the fiberoptic light phototherapy pad currently supplied commercially and include woven layers of emitting optical fibers 134 woven only in the warp direction and fill threads 136 interwoven therewith in the weft direction. As is conventional in the weaving art, weft threads are normally carried by the shuttle of the weaving loom while the warp threads extend length-wise of the loom, crossed by the weft threads.

As noted in the Daniel patent, by bending the emitting optical fibers 134 at predetermined intervals around the fill threads 136, the light couples out at locations known as scattering centres 138 and which light is directed toward the infant. Thus layers 122-130 emit light radiation toward the patient.

In the embodiment shown, however light detecting layer 132 which is facing the infant, does not emit light radiation but instead detects light radiation. In light detecting layer 130, therefore, light is collected by detecting optical fibers 140, again, by providing bending by means of fill threads 142 interwoven among the detecting optical fibers 140. Light is thus absorbed or collected by the detecting optical fibers 140 and is transmitted by a optical fiber (96 of Figure 4) to the optical transducer 98.

To complete the construction, one or both ends of the emitting optical fibers 134 may be bundled together and formed into a plug or fiber bundle that eventually forms, or communicates with optical fiber 86 of Figure 4 and likewise, the detecting optical fibers 140 are formed into a plug or bundled to form or communicate with optical fiber 96 of Figure 4. The light emitting layer 122, furthermost from the infant may also have a reflective coating 144 to direct scattered light toward the infant.

Thus the multilayer optical pad contains multiple layers of emitting layers, as does the commercial product, however the final layer prior to the patient is a detecting layer. The overall construction of the detecting layer is similar to the emitting layer, that is, all of the layers are woven in the same manner; the difference being that the light emitting layers 122-130 are supplied light by the laser 78 (Figure 4) and emit light radiation toward the infant while the light detecting layer 132 collects representative light and transmits that light back to optical transducer 98.

Accordingly, the light detecting layer 132 is relatively transparent and allows light to pass through to the infant while collecting enough light to represent the light density of the light emitting layers 122-130.

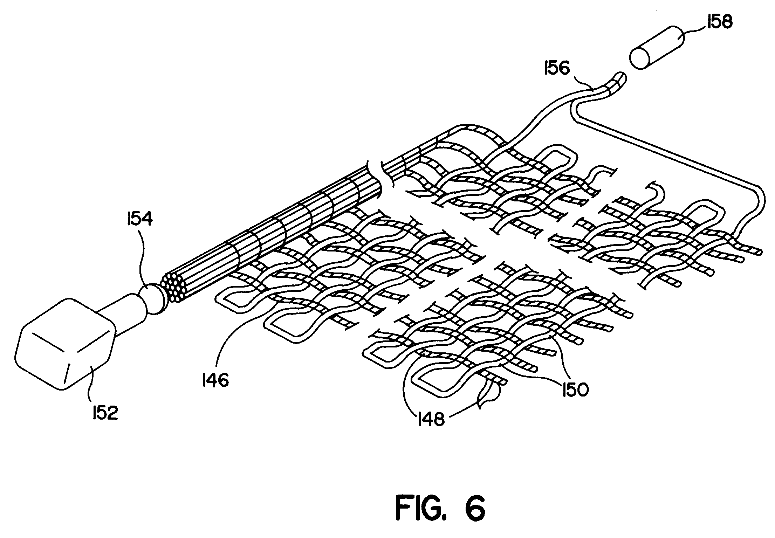

Turning now to Figure 6, there is shown a schematic view of yet another light sensor that can be used with the present invention. In particular, in the Figure 6 embodiment, there is a combined a single unitary layer 146 and which is formed by emitting warp fibers 148 woven with detecting weft fibers 150. A connector 152 is formed at the ends of the emitting warp fibers 148 and which connector 152 is adapted to be connected to the laser source of light radiation transmitted through optical fiber 86 of Figure 4. Thus connector 152 receives the light radiation from the laser source and transmits that light radiation through fiber bundle 154 to the individual emitting warp fibers 148 to provide phototherapy to an infant.

In similar fashion, the detecting weft fibers 150 are formed of optical fibers and which detect light emitted from the emitting warp fibers 148. Detecting weft fibers 150 thus transmit the detected light through fiber bundle 156 to a connector 158 for connection to or other communication with the optical fiber 96 of Figure 4.

Thus the woven fiber unitary layer of Figure 6 can be woven as generally described in Daniel, and is both an emitter of light radiation to the infant and a detector of light radiation thus emitted and which reaches the infant. The unitary layer 146 is therefore usable as a light sensor for use with the present invention.

## Claims

1. A phototherapy system for providing light radiation to an infant having at least one fiberoptic pad (22) adapted to be placed near the infant to allow light emitted from the pad (22) to impinge upon the patient, a light source (16) for emitting light radiation and a fiberoptic coupling means (18) for transmitting light from said light source (16) to said fiberoptic pad 22, characterised in that said light source (16) comprises a laser (16) producing a monochromatic light at a known wavelength.

2. A system for providing phototherapy to infants, said system being characterised by:
a) a laser (78) providing coherent light radiation at a predetermined wavelength;
b) a plurality of fiberoptic light pads (88) adapted to be placed near the infant;
c) a plurality of optical fibers (86) connecting said laser (78) to each of said plurality of fiberoptic light pads (88) such that the light radiation from said laser (78) is transmitted outwardly from said fiberoptic light pads (88) to impinge upon the infants;
d) light sensors (94) for detecting the intensity of light emitted by each of said fiberoptic light pads (88) toward the infants and to produce an electrical signal representing such light intensities; and
e) control means (104) adapted to respond to the electrical signal from each of said light sensors (94) to control the intensity of light transmitted individually to each of said plurality of fiberoptic pads (88) to thereby control the intensity of light impinging upon the infants from the fiberoptic pads (88) for providing phototherapy.

3. A system for providing phototherapy to infants as claimed in Claim 2, comprising transducer means (98) for receiving the light signals from said light sensors (94) and to transmit electrical signals indicative of such light signals; input means (106) to receive information from a user and to produce an electrical signal indicative of the amount of light radiation desired to be emitted from each of said fiberoptic light pads (88) to impinge upon an infant; and a central processing unit (104) receiving signals from said transducer means (98) and said input means (106), said central processing unit (104) comparing said signals to control the light emitted from each of said fiberoptic light pads (88) in accordance with the amount of light radiation desired for each infant.

4. A system as claimed in Claim 3, including a variable attenuator (92) adapted to receive signals from said central processing unit (104) to control the amount of light radiation impinging upon an infant.

5. A system as claimed in Claim 3 or 4, wherein said input means (106) produces an electrical signal indicative of the light intensity to impinge upon an infant from a fiberoptic light pad (88).

6. A system as claimed in any one of Claims 2 to 5, wherein the light sensors (94) are located adjacent each of said plurality of fiberoptic pads (88) to sense the intensity of light radiation emitted from said fiberoptic pads (88) to impinge upon the infants.

7. A control system as claimed in any one of Claims 2 to 6, wherein said means (104) to control the intensity of the light output from said laser (78) comprises means to control the electrical power (120) to said laser (78).

8. A method of providing light therapy to an infant characterised by the steps of:
a) providing a light emitting fiberoptic pad;
b) locating the fiberoptic pad adjacent an infant such that light eminating from the pad impinges upon the infant;
c) directing light radiation from a laser into the fiberoptic pad to cause it to emit light radiation of a predetermined monochromatic wavelength upon the infant.

9. A method of providing light radiation to a plurality of infants for phototherapy, characterised by the steps of:
a) providing a plurality of fiberoptic woven phototherapy pads;
b) placing each of said plurality of phototherapy pads in position in close proximity to an infant;
c) directing light from a single laser having a known monochromatic wavelength into each of said plurality of phototherapy pads to cause light radiation to be emitted from each of the phototherapy pads to impinge upon the infants;
d) sensing the intensity of the light radiation emitted from each of said plurality of phototherapy pads and impinging upon the infants; and
e) controlling the intensity of the light emitted from each of said plurality of phototherapy pads in response to the intensity sensed in step d).
